(19) Europäisches Patentamt
European Patent Office
Office européen des brevets

(11) Veröffentlichungsnummer: **0 173 943**
**A1**

# (12) EUROPÄISCHE PATENTANMELDUNG

(21) Anmeldenummer: **85110718.5**

(22) Anmeldetag: **26.08.85**

(51) Int. Cl.⁴: **C 07 D 401/06**
**C 07 D 401/14, A 61 K 31/44**

(30) Priorität: **05.09.84 DE 3432563**

(43) Veröffentlichungstag der Anmeldung:
**12.03.86 Patentblatt 86/11**

(84) Benannte Vertragsstaaten:
**AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Rosentreter, Ulrich, Dr.**
**Kondorweg 23**
**D-5600 Wuppertal 1(DE)**

(72) Erfinder: **Perzborn, Elisabeth, Dr.**
**Am Tescherbusch 13**
**D-5600 Wuppertal 11(DE)**

(72) Erfinder: **Seuter, Friedel, Dr.**
**Moospfad 16**
**D-5600 Wuppertal 1(DE)**

(54) **Neue Pyridylethyl-Dihydropyridine, Verfahren zu ihrer Herstellung und ihre Verwendung in Arzneimitteln.**

(57) Die Erfindung betrifft neue Pyridylethyl-Dihydropyridine der allgemeinen Formel (I)

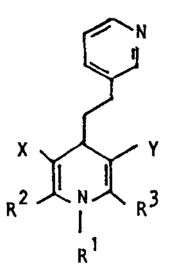

(I)

in welcher die Substituenten $R^1$, $R^2$, $R^3$, X, Y und N die in der Beschreibung angegebene Bedeutung haben, mehrere Verfahren zu ihrer Herstellung sowie ihre Verwendung in Arzneimitteln, insbesondere zur Bekämpfung von thromboembolischen und ischämischen Erkrankungen.

BAYER AKTIENGESELLSCHAFT 5090 Leverkusen, Bayerwerk
Konzernverwaltung RP
Patentabteilung KS/bo/c

Neue Pyridylethyl-Dihydropyridine, Verfahren zu ihrer Herstellung und ihre Verwendung in Arzneimitteln

Die Erfindung betrifft neue Pyridylethyl-Dihydropyridine, mehrere Verfahren zu ihrer Herstellung sowie ihre Verwendung in Arzneimitteln, insbesondere zur Bekämpfung von thromboembolischen und ischämischen Erkrankungen.

Es ist bereits bekannt, daß 1,4-Dihydropyridine interessante pharmakologische Eigenschaften besitzen (F. Bossert, W. Vater, Die Naturwissenschaften 58, (11), 578 (1971)).

Die vorliegende Erfindung betrifft neue Pyridylethyl-Dihydropyridine der allgemeinen Formel (I)

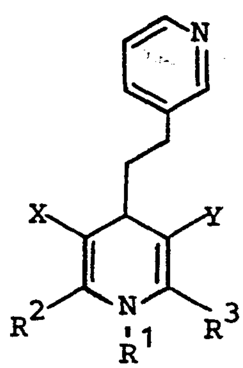

(I)

in welcher

$R^1$ - für Wasserstoff oder für geradkettiges oder verzweigtes Alkyl (bevorzugt mit bis zu 6, besonders bevorzugt mit bis zu 4 C-Atomen), gegebenenfalls substituiert durch Halogen (bevorzugt Fluor, Chlor, Brom), Nitro, Cyan, Carboxy, Carbonamido, Alkoxy, Alkoxycarbonyl (jeweils bevorzugt mit bis zu 6, besonders bevorzugt mit bis zu 4 C-Atomen), steht,

$R^2$,$R^3$ - gleich oder verschieden sind und für Wasserstoff, für geradkettiges oder verzweigtes Alkyl (bevorzugt mit bis zu 6, besonders bevorzugt mit bis zu 4 C-Atomen), gegebenenfalls substituiert durch Halogen (bevorzugt Fluor, Chlor, Brom), Aryl (bevorzugt mit 6 - 10 C-Atomen, besonders bevorzugt Phenyl), Carboxy, Alkoxy, Alkoxycarbonyl (jeweils bevorzugt mit bis zu 6, besonders bevorzugt mit bis zu 4 C-Atomen), stehen oder mit X bzw. Y durch eine Einfachbindung zu einem 5- bis 6-gliedrigen Ring verbunden sind,

X,Y - gleich oder verschieden sind und für Wasserstoff, für Cyan oder für die Gruppe $-\underset{\underset{O}{\|}}{C}-Z-R^4$ stehen,

wobei

Z - für eine direkte Bindung oder für Sauerstoff, Schwefel oder NH steht,

und

$R^4$ - für Wasserstoff oder für geradkettiges oder verzweigtes Alkyl (bevorzugt mit bis zu 6, besonders bevorzugt mit bis zu 4 C-Atomen), gegebenenfalls substituiert durch Halogen (bevorzugt Fluor, Chlor, Brom), Nitro, Pyridyl, Cyan, Amino, Carboxy, Alkoxy, Alkoxycarbonyl (bevorzugt jeweils mit bis zu 6, besonders bevorzugt mit bis zu 4 C-Atomen), steht,

sowie deren physiologisch unbedenklichen Salze.

Bevorzugte Verbindungen der Formel (I) sind solche, in welchen

$R^1$ - für Wasserstoff oder für geradkettiges oder verzweigtes Alkyl mit bis zu 6 C-Atomen, gegebenenfalls substituiert durch Fluor, Chlor, Brom, Carboxy, Alkoxy oder Alkoxycarbonyl mit jeweils bis zu 6 C-Atomen, steht,

$R^2$,$R^3$ - gleich oder verschieden sind und für Wasserstoff oder für geradkettiges oder verzweigtes Alkyl mit bis zu 6 C-Atomen, gegebenenfalls substituiert durch Fluor, Chlor, Brom, Aryl mit 6 bis 10 C-Atomen, Carboxy, Alkoxy, Alkoxycarbonyl mit jeweils bis zu 6 C-Atomen, stehen, oder mit X bzw. Y durch eine Einfachbindung zu einem 5- bis 6-gliedrigen Ring verbunden sind,

X,Y - gleich oder verschieden sind und für Wasserstoff, für Cyan oder für die Gruppe $-\underset{O}{\overset{\|}{C}}-Z-R^4$ stehen,

wobei

Z - für eine direkte Bindung oder für Sauerstoff, Schwefel oder NH steht,

und

$R^4$ - für Wasserstoff oder für geradkettiges oder verzweigtes Alkyl mit bis zu 6 C-Atomen, gegebenenfalls substituiert durch Fluor, Chlor, Brom, Nitro, Pyridyl, Cyan, Amino, Carboxy, Alkoxy, Alkoxycarbonyl mit jeweils 6 C-Atomen, steht,

sowie deren physiologisch unbedenklichen Salze.

Besonders bevorzugte Verbindungen der Formel (I) sind solche, in welchen

$R^1$ - für Wasserstoff oder für geradkettiges oder verzweigtes Alkyl mit bis zu 4 C-Atomen, gegebenenfalls substituiert durch Carboxy, Alkoxy, Alkoxycarbonyl mit jeweils bis zu 4 C-Atomen, steht,

$R^2$,$R^3$ - gleich oder verschieden sind und für Wasserstoff oder für geradkettiges oder verzweigtes Alkyl mit bis zu 4 C-Atomen, gegebenenfalls substituiert durch Fluor, Chlor, Brom, Phenyl, Carboxy, Alkoxycarbonyl mit bis zu 4 C-Atomen, stehen, oder mit X bzw. Y durch eine Einfachbindung zu einem 5- bis 6-gliedrigen Ring verbunden sind,

X,Y - gleich oder verschieden sind und für Wasserstoff, für Cyan oder für die Gruppe $-\underset{\overset{\|}{O}}{C}-Z-R^4$ stehen,

wobei

Z - für eine direkte Bindung oder für Sauerstoff oder NH steht,

und

$R^4$ - für Wasserstoff oder für geradkettiges oder verzweigtes Alkyl mit bis zu 4 C-Atomen, gegebenenfalls substituiert durch Fluor, Chlor, Brom, Cyan, Pyridyl, Amino, Carboxy, Alkoxy, Alkoxycarbonyl mit jeweils bis zu 4 C-Atomen steht,

sowie deren physiologisch unbedenklichen Salze.

Als Salze sind beispielsweise Hydrochloride, Hydrogensulfate, Sulfate, Hydrogenphosphate, Acetate, Maleate, Benzoate, Tartrate, Citrate, Lactate, Fumarate, Formiate oder Succinate zu nennen.

Die erfindungsgemäßen Verbindungen der allgemeinen Formel (I), in der

$R^1$ - $R^4$, X, Y, Z die oben angegebene Bedeutung haben,

können hergestellt werden, indem man

(A) Enamine der allgemeinen Formel (II)

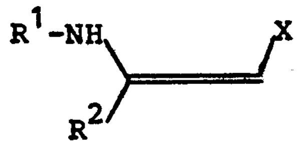

(II),

in welcher

$R^1$, $R^2$, X die oben angegebene Bedeutung haben,

X und $R^4$ jedoch nicht für Wasserstoff stehen,

mit Ketoverbindungen der allgemeinen Formel (III)

$$R^3-\underset{\overset{\parallel}{O}}{C}-CH_2-Y \qquad \text{(III)}$$

in welcher

$R^3$, Y die oben angegebene Bedeutung haben,

Y und $R^4$ jedoch nicht für Wasserstoff stehen,

und dem Aldehyd der Formel (IV)

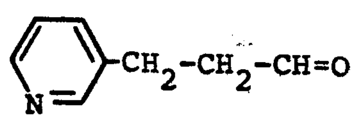

(IV)

gegebenenfalls in Gegenwart eines inerten organischen Lösungsmittels umsetzt,

oder indem man

(B) Ketoverbindungen der allgemeinen Formel III (a, b)

$$R^2-\underset{\underset{O}{\|}}{C}-CH_2-X \quad (IIIa), \qquad R^3-\underset{\underset{O}{\|}}{C}-CH_2-Y \quad (IIIb)$$

gegebenenfalls in Form ihrer Mischungen zu gleichen Teilen (falls $R^3$, $R^2$ und/oder X, Y verschieden),

in welchen

$R^2$, $R^3$, X, Y die angegebene Bedeutung haben,

X, Y und $R^4$ jedoch nicht für Wasserstoff stehen,

mit dem Aldehyd der Formel (IV) und einem Amin der allgemeinen Formel (V)

$$R^1-NH_2 \qquad (V)$$

in welcher

$R^1$ die oben angegebene Bedeutung hat, bzw. dessen Salz,

gegebenenfalls in Gegenwart eines inerten organischen Lösungsmittels umsetzt,

und gegebenenfalls Verbindungen der allgemeinen Formel (I), in welchen die Reste $R^1$, $R^2$, $R^3$, X

und/oder Y Alkoxycarbonylgruppierungen enthalten und/oder in welchen X oder Y selbst eine Alkoxycarbonylgruppe ist, nach üblichen Methoden verseift und anschließend gegebenenfalls solche, in denen X oder Y für Carboxyl steht, decarboxyliert.

Verwendet man nach Verfahrensvariante (A) 3-Amino-2-cyclohexenon, 1,3-Cyclohexandion und 3-(3-Pyridyl)-propionaldehyd als Ausgangsstoffe, so kann man die Reaktion durch folgendes Schema darstellen:

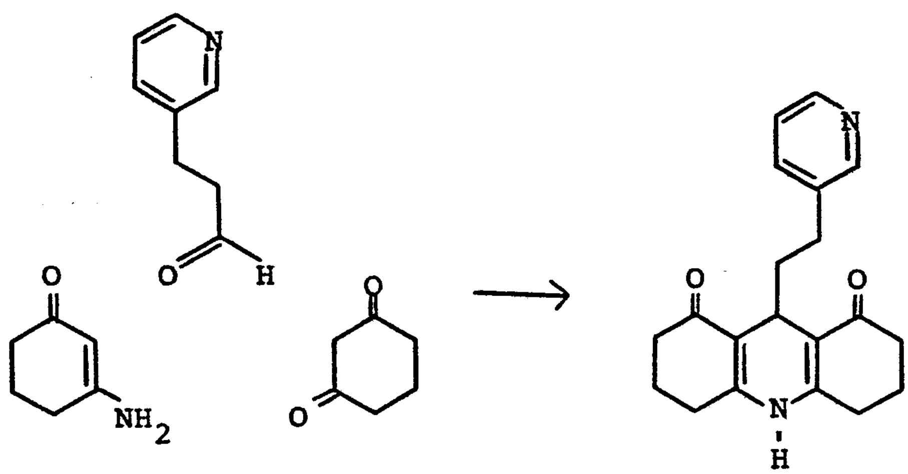

Verwendet man nach Verfahrensvariante (B) Acetessigsäuremethylester, 3-(3-Pyridyl)-propionaldehyd und Methylaminhydrochlorid als Ausgangsstoffe, so kann man die Reaktion durch folgendes Schema darstellen:

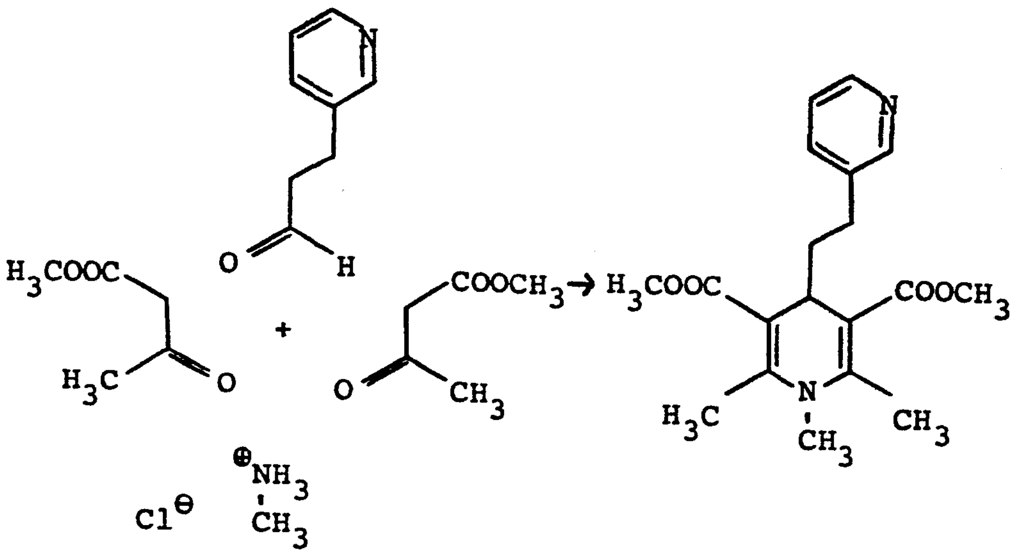

Die als Ausgangsstoffe verwendeten Enamine (II) sind bekannt oder können nach literaturbekannten Methoden hergestellt werden (vgl. A.C. Cope, J. Amer. Chem. Soc. 67, 1017 (1945)).

Die als Ausgangsstoffe verwendeten Ketoverbindungen (III) sind bekannt oder können nach literaturbekannten Methoden hergestellt werden (vgl. D. Borrmann "Umsetzung von Diketen mit Alkoholen, Phenolen und Mercaptanen" in Houben-Weyl, Methoden der organischen Chemie, Band VII/4, 230 - (1968); Y. Oikana, K. Sugano, O. Yonemitsu, J. org. Chem. 43, 2087/8 (1978)).

Das erfindungsgemäß verwendete Aldehyd der Formel (IV) kann nach einem bekannten Verfahren hergestellt werden (vgl. A.J. Mancuso, D.S. Brownfain, D. Swern, J. org. Chem. 44, 4148 (1979)).

Die erfindungsgemäß verwendeten Amine der Formel (V) bzw. deren Salze sind bekannt.

Als Lösungsmittel kommen alle inerten organischen Lösungsmittel in Frage. Hierzu gehören vorzugsweise Alkohole wie Methanol, Ethanol, Propanol, Isopropanol, Ether wie Diethylether, Dioxan, Tetrahydrofuran, Glykolmonomethylether, Glykoldimethylether, Eisessig, Acetonitril, Dimethylformamid, Dimethylsulfoxid, Hexamethylphosphorsäuretriamid oder Pyridin.

Die Überführung des Alkoxycarbonyls in die Carboxygruppe kann nach den üblichen Methoden der Hydrolyse durchgeführt werden. Bevorzugt werden die üblichen Basen wie Alkali- oder Erdalkalihydroxide, insbesondere Lithium-, Natrium-, Kalium-, Calcium- oder Bariumhydroxid verwendet.

Die Decarboxylierung kann nach üblichen Methoden erfolgen, vorzugsweise in saurem Medium, insbesondere in Salzsäure, Schwefelsäure, Phosphorsäure, Perchlorsäure oder Trifluoressigsäure unterschiedlicher Konzentrationen.

Die Reaktionstemperaturen können in einem breiten Bereich variiert werden. Im allgemeinen arbeitet man in einem Bereich von 10°C bis 150°C, bevorzugt in einem Bereich von 20°C bis 100°C. Gegebenenfalls kann es vorteilhaft sein, beim Siedepunkt des Lösungsmittels zu arbeiten.

Das Mengenverhältnis der Reaktanten zueinander ist beliebig, im allgemeinen werden bei Verfahren (A) Enamin (II), Ketoverbindung (III) und Aldehyd (IV) im Verhältnis 1:1:1, bei Verfahren (B) Ketoverbindung (III), Aldehyd (IV) und Amin (V) bzw. sein Salz im Verhältnis 2:1:1 eingesetzt. Es hat sich jedoch als zweckmäßig erwiesen, im Verfahren (B) das Amin im bis zu 2-fachen Überschuß zuzugeben.

Die vorstehenden Herstellungsverfahren sind lediglich zur Verdeutlichung angegeben, die Herstellung der Verbindungen der Formel (I) ist nicht auf diese Verfahren beschränkt, sondern jede Modifikation der Verfahren ist in üblicher Weise für die Herstellung der erfindungsgemäßen Verbindungen (I) anwendbar.

Die vorliegende Erfindung betrifft auch die Verwendung der erfindungsgemäßen Stoffe der Formel (I) als Hemmer/Stimulatoren von enzymatischen Reaktionen im Rahmen des Arachidonsäurestoffwechsels. Derartige Substanzen sind zur Verhütung und Behandlung von Erkrankungen der Atemwege wie Emphysem, Schocklunge, pulmonaler Hypertonie, Ödem, Thrombose und Thromboembolie, Ischämien (periphere, cardiale, cerebrale Durchblutungsstörungen), Herz- und Hirninfarkte, Herzrhythmusstörungen, Angina pectoris, Hypertonie sowie Arteriosklerose geeignet.

Die erfindungsgemäßen Stoffe sind bevorzugt Hemmer der Thromboxansynthese und stimulieren gleichzeitig die Prostacyclinsynthese.

Die neuen Wirkstoffe können in bekannter Weise in die üblichen Formulierungen (wie z.B. Tabletten, Kapseln, Dragees, Pillen, Granulate, Cremes, Suppositorien, Emulsionen, Suspensionen sowie Infusions- und Injektionslösungen) unter Verwendung inerter, nichttoxischer, pharmazeutisch geeigneter Trägerstoffe oder Lösungsmittel übergeführt werden.

Besonders geeignet sind Formulierungen, die etwa 0,1 - 10 Gewichtsprozent an Wirkstoff enthalten, vorzugsweise wäßrige Lösungen. Besonders bevorzugt sind wäßrige Lösungen mit einem pH-Wert zwischen 6 und 8.

Die Formulierungen erfolgen nach den üblichen Methoden, beispielsweise durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel wie Paraffine (z.B. Erdölfraktionen), pflanzl. Öle (z.B. Erdnuß-/Sesamöl), Alkohole (z.B. Ethylalkohol, Glyzerin), Glykole (z.B. Propylenglykol, Polyethylenglykol), feste Trägerstoffe wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-, Traubenzucker), Emulgiermittel (z.B. Polyoxyethylen-Fettsäureester, Polyoxyethylen-Fettalkoholether, Alkylsulfonate, Arylsulfonate), Dispergiermittel (z.B. Lignin-Sulfitablaugen, Methylcellulose, Stärke, Polyvinylpyrrolidon) und Gleitmittel (z.B. Magnesiumstearat, Talkum, Stearinsäure, Natriumlaurylsulfat).

Die Applikation erfolgt in üblicher Weise je nach Bedarf lokal, oral oder parenteral.

Die Dosierung erfolgt im allgemeinen in einem Bereich von 0,05 - 100 mg/kg Körpergewicht, insbesondere von 0,1 - 50 mg/kg Körpergewicht.

Im Unterschied zu Indometacin, welches die Prostaglandinsynthese aus der Arachidonsäure insgesamt hemmt, greifen die erfindungsgemäß einzusetzenden Wirkstoffe viel spezifischer in den Stoffwechsel der für die Bildung des Prostacyclins ($PGI_2$), des Thromboxans maßgebende Enzyme ein, so daß nicht nur der schädigende, gefäßverengende und arrythmienerhöhende Einfluß des Thromboxans reduziert, sondern auch der gefäßerweiternde Einfluß des $PGI_2$'s durch Stimulierung seiner Bildung erhöht wird.

Die biologische Wirkung der erfindungsgemäß hergestellten Verbindungen wurde durch folgende Experimente nachgewiesen:

I. <u>$^{3}$H-Arachidonsäuremetabolismus</u>

Der Arachidonsäuremetabolismus in Human-Thrombozyten wurde mit Hilfe von Tritium-markierter Arachidonsäure

untersucht. Die Thrombozyten metabolisieren die Arachidonsäure über den Cyclooxygenaseweg zu $TXA_2$ und HHT und über den Lipogenaseweg zu 12-HETE, die dünnschichtchromatographisch getrennt werden können (vgl. Bailey, J.M. et al., Prostaglandins 13, 479 - 492, 1977). Inhibitoren der einzelnen enzymatischen Reaktionen verändern das chromatographische Verteilungsmuster in charakteristischer Weise.

Gewaschene Human-Thrombozyten von gesunden Spendern, die 14 Tage kein Medikament eingenommen hatten, wurden mit Prüfsubstanz 2 Min. bei 37°C inkubiert und anschließend mit $^3H$-Arachidonsäure weitere 10 Min. bei 37°C inkubiert. Die Suspension wurde angesäuert und mit Essigester extrahiert. Der Essigester wurde unter Stickstoffatmosphäre abgedampft und der Rückstand in Methanol/Trichlormethan (1:1) aufgenommen und auf DC-Plastikfolien aufgetragen. Die Trennung erfolgte mit einem Fließmittelgemisch Trichlormethan/Methanol/Eisessig/Wasser (80:8:1:0,8). Die Verteilung der Radioaktivität wurde mittels eines Radioscanners gemessen.

II. Prostacyclinstimulation

Außerdem stimulieren die erfindungsgemäß zu verwendenden 1,4-Dihydropyridine die Synthese von $PGI_2$. $PGI_2$ wirkt im Gegensatz zu dem vasokonstriktorischen und thrombozytenaggregationsauslösenden Thromboxan gefäßdilatierend und thrombozytenaggregationshemmend.

Stimulation im Vollblut

Im Vollblut läßt sich durch Kollagen die Bildung von $PGI_2$ induzieren. Die in Thrombozyten gebildeten Endoperoxide werden wahrscheinlich durch Leukozyten-Lipoxygenase in $PGI_2$ umgewandelt. Das stabile Endprodukt der $PGI_2$-Umwandlung, 6-Keto-$PGF_{1\alpha}$, wird radioimmunologisch bestimmt.

III. Thrombozytenaggregation

Thrombozyten und deren Adhäsion - sowie Aggregationsfähigkeit - sind, besonders im arteriellen Schenkel des Gefäßsystems, ein wesentlicher pathogenetischer Faktor bei der Entstehung von Thrombosen. Überraschenderweise waren die erfindungsgemäßen Verbindungen auch in diesem Test wirksam, was die Substanz in Verbindung mit den anderen Eigenschaften als besonders günstig erscheinen läßt.

Für die in vitro-Versuche wurde Blut von gesunden Probanden beiderlei Geschlechts verwendet. Als Antikoagulans wurde ein Teil 3,8-prozentige Natriumzitratlösung 9 Teilen Blut zugemischt.

Zu 0,95 ml Blut wurden 0,025 ml Prüflösung (bzw. Lösungsmittel als Kontrolle) gegeben und 10 Minuten bei 37°C im Heizblock des Aggregometers vorinkubiert. Anschließend wurde die Thrombozytenaggregation nach der Impedanzmethode bei 37°C bestimmt. Hierzu wurde die vorinkubierte Probe mit 0,025 ml einer Kollagensuspension, dem aggregationsauslösenden Agens, versetzt. Die Veränderung des

elektrischen Widerstandes zwischen zwei Elektroden in der gerührten Probe ist Folge des Aggregationsgrades der Thrombozyten und wird während einer Zeitdauer von 8 Minuten aufgezeichnet. Die Hemmung wird halbquantitativ in Relation zum Verlauf der Kontrollkurven bestimmt.

Als Grenzkonzentration wird der Bereich der minimal effektiven Konzentration angegeben.

Tabelle 1

Hemmung der $TXA_2$-Synthese

| Beispiel-Nr. | Grenzkonzentration für Hemmung [mg/l] |
|---|---|
| 1 | 10-1 |
| 3 | 3-0,3 |
| 7 | 10-1 |
| 10 | 10-1 |
| 11 | 10-1 |
| 15 | 10-1 |
| 20 | 1-0,3 |

Tabelle 2

Hemmung der Thrombozytenaggregation

| Beispiel-Nr. | Grenzkonzentration für Hemmung [mg/l] |
|---|---|
| 1 | 10-3 |
| 3 | 10-3 |
| 4 | 10-3 |
| 6 | 10-3 |
| 8 | 10-3 |

Tabelle 2 (Fortsetzung)

| Beispiel-Nr. | Grenzkonzentration für Hemmung [mg/l] |
|---|---|
| 13 | 10-3 |
| 15 | 10-3 |
| 17 | 10-3 |

Herstellungsbeispiele

Beispiel 1

1,4-Dihydro-2,6-dimethyl-4-/2-(3-pyridyl)ethyl/-pyridin-3,5-dicarbonsäuredimethylester

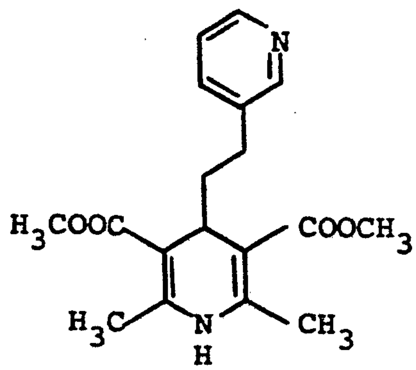

2,5 g 3-(3-Pyridyl)propionaldehyd werden zusammen mit 2 ml Acetessigsäuremethylester und 2,1 g 3-Aminocrotonsäuremethylester in 15 ml Ethanol 30 Minuten unter Rückfluß erhitzt. Nach dem Abkühlen werden die ausgefallenen Kristalle abgesaugt und im Vakuum getrocknet.

Ausbeute: 2,0 g (48 % d.Th.) Fp.: 207 - 209°C

Beispiel 2

1,4-Dihydro-2,6-dimethyl-4-/2-(3-pyridyl)ethyl/-pyridin-3,5-dicarbonsäure-3-(2-cyanoethyl)-5-methylester

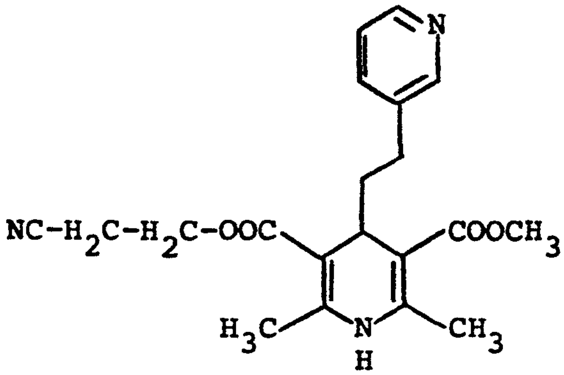

2 g 3-(3-Pyridyl)propionaldehyd werden zusammen mit 1,6 ml Acetessigsäuremethylester und 2 g 3-Aminocrotonsäure-(2-cyano)ethylester in 10 ml Ethanol 2 h unter Rückluß erhitzt. Nach dem Einengen kristallisiert das Produkt aus einem Gemisch von Essigester/Ether/Ethanol.

Ausbeute: 3,4 g (63 % d.Th.) Fp.: 126 - 129°C.

Beispiel 3

1,4-Dihydro-2,6-dimethyl-4-/2-(3-pyridyl)ethyl7-pyridin-3,5-dicarbonsäure-3-/3-(3-pyridyl)propyl]-5-methylester

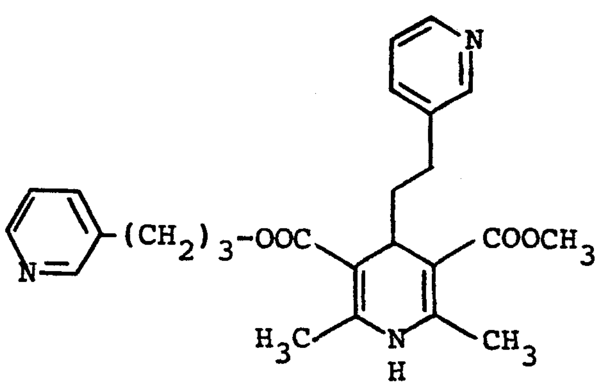

11 g Acetessigsäure-[3-(3-pyridyl)]propylester werden zusammen mit 6,75 g 3-(3-Pyridyl)propionaldehyd und 7 g 3-Aminocrotonsäuremethylester in 50 ml Ethanol 2 h unter Rückfluß erhitzt. Nach dem Eindampfen kristallisiert das Produkt aus Ether.

Ausbeute: 17,5 g (80 % d.Th.) Fp.: 147 - 150°C.

Beispiel 4

1,4-Dihydro-2-methoxycarbonylmethyl-6-methyl-4-[2-(3-pyridyl)ethyl]-pyridin-3,5-dicarbonsäuredimethylester

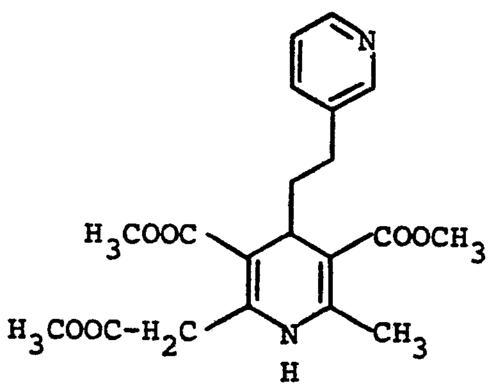

1,35 g 3-(3-Pyridyl)propionaldehyd werden zusammen mit 1,45 ml Acetondicarbonsäuredimethylester und 1,15 g 3-Aminocrotonsäuremethylester 2 h in 10 ml Ethanol unter Rückfluß erhitzt. Nach dem Einengen wird der ölige Rückstand an Kieselgel mit Toluol:Essigester = 6:4 als Elutionsmittel chromatographiert. Man erhält so drei Fraktionen, von denen die letzte nach dem Eindampfen 0,8 g kristallines Produkt liefert.

Ausbeute: 0,8 g (20 % d.Th.) Fp.: 129 - 130°C.

Beispiel 5

1,4-Dihydro-2,6-dimethyl-4-[2-(3-pyridyl)ethyl]-pyridin-3,5-dicarbonsäurediethylester

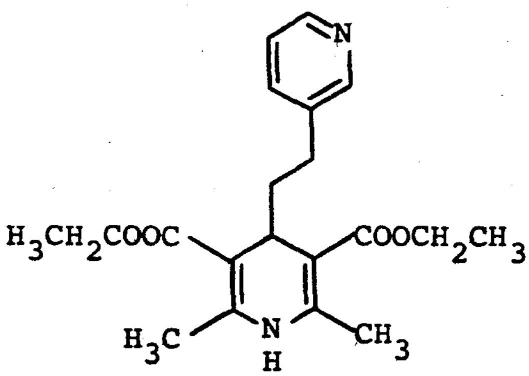

2 g 3-(3-Pyridyl)propionaldehyd werden zusammen mit 1,95 g Acetessigsäureethylester und 1,94 g 3-Aminocrotonsäureethylester in 20 ml Ethanol 1 h unter Rückfluß erhitzt. Nach dem Abkühlen auf 0°C werden die ausgefallenen Kristalle abgesaugt und im Vakuum getrocknet.

Ausbeute: 2,8 g (52 % d.Th.) Fp.: 153 - 155°C.

Beispiel 6

1,2,3,4,5,6,7,8,9,10-Decahydro-9-[2-(3-pyridyl)ethyl]-acridin-1,8-dion

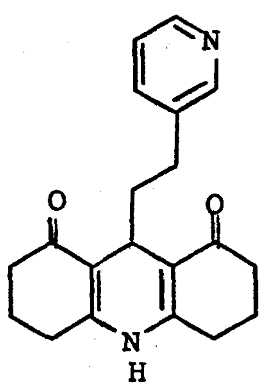

2 g 3-(3-Pyridyl)propionaldehyd werden zusammen mit 1,68 g 1,3-Cyclohexandion und 1,67 g 3-Amino-2-cyclohexen-1-on in 20 ml Ethanol 1 h unter Rückfluß erhitzt. Nach dem Einengen wird der Rückstand in Methylenchlorid aufgenommen und mit verdünnter Schwefelsäure bei einem pH-Wert von 3 extrahiert. Die wäßrige Phase wird auf einen pH-Wert von 8 gebracht und 3 mal mit Essigester extrahiert. Die vereinigten Essigesterphasen werden mit Magnesiumsulfat getrocknet und eingedampft. Der Rückstand kristallisiert aus Ether.

Ausbeute: 1,5 g (31 % d.Th.) Fp.: 205 - 210°C.

Beispiel 7

1,4-Dihydro-2,6-dimethyl-4-/2-(3-pyridyl)ethyl/-pyridin-3,5-dicarbonsäure-3-isopropyl-5-methylester

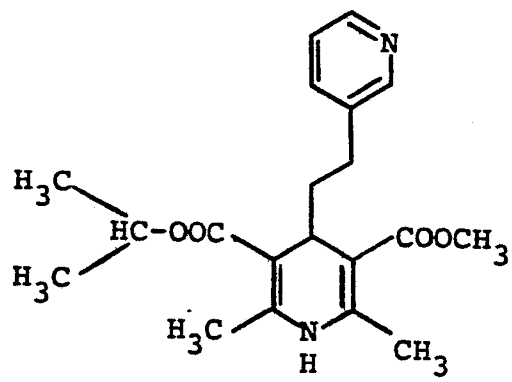

2 g 3-(3-Pyridyl)propionaldehyd werden zusammen mit 2,16 g Acetessigsäureisopropylester und 1,72 g 3-Aminocrotonsäuremethylester in 20 ml Ethanol 1 h unter Rückfluß erhitzt. Die Aufarbeitung erfolgt analog zu Beispiel 6.

Ausbeute: 0,92 g (17 % d.Th.) Fp.: 163 - 173°C.

0173943

**Beispiel 8**

1,4,5,6,7,8-Hexahydro-2-methyl-4-/2-(3-pyridyl)ethyl7-chinolin-5-on-3-carbonsäuremethylester

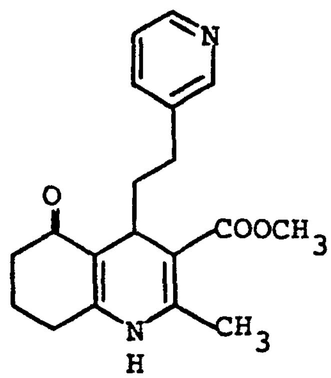

2 g 3-(3-Pyridyl)propionaldehyd werden zusammen mit 1,74 g Acetessigsäuremethylester und 1,67 g 3-Amino-2-cyclohexen-1-on in 20 ml Ethanol 1 h unter Rückfluß erhitzt. Nach Abkühlen auf 0°C werden die ausgefallenen Kristalle abgesaugt und im Vakuum getrocknet.

Ausbeute: 1,75 g (36 % d.Th.) Fp.: 184 - 186°C.

**Beispiel 9**

3,5-Diacetyl-1,4-dihydro-2,6-dimethyl-4-/2-(3-pyridyl)-ethyl7-pyridin

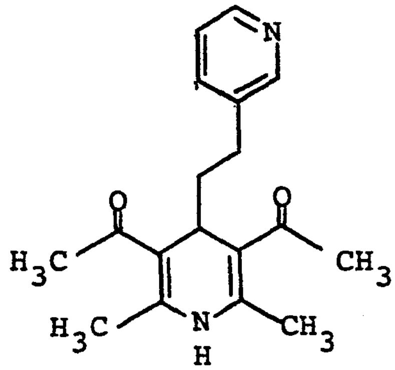

2 g 3-(3-Pyridyl)propionaldehyd werden zusammen mit 1,49 g 4-Amino-3-penten-2-on und 1,5 g 2,4-Pentandion 3 h unter Rückfluß erhitzt. Nach Abkühlen auf 0°C saugt man die ausgefallenen Kristalle ab und trocknet im Vakuum.

Ausbeute: 1,9 g (42,5 % d.Th.)    Fp.: 122 - 124°C (Zers.)

Beispiel 10

5-Cyano-1,4-dihydro-2,6-dimethyl-4-[2-(3-pyridyl)ethyl]-pyridin-3-carbonsäuremethylester

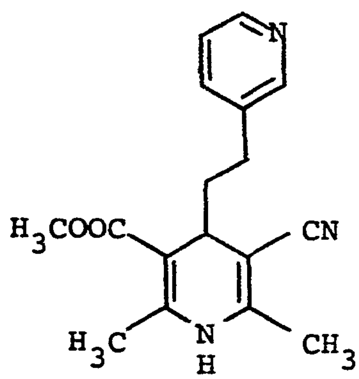

2 g 3-(3-Pyridyl)propionaldehyd werden zusammen mit 1,23 g 3-Aminocrotonsäurenitril und 1,74 g Acetessigsäuremethylester 3 h unter Rückfluß erhitzt. Nach Abkühlen auf 0°C saugt man die ausgefallenen Kristalle ab und trocknet im Vakuum.

Ausbeute: 2,2 g (49 % d.Th.)    Fp.: 151 - 153°C.

Beispiel 11

1,4-Dihydro-2,6-dimethyl-4-[2-(3-pyridyl)ethyl]-pyridin-3,5-dicarbonsäure-3-methylester-5-amid

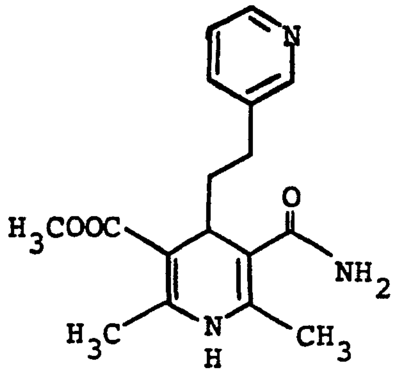

2 g 3-(3-Pyridyl)propionaldehyd werden zusammen mit 1,5 g 3-Aminocrotonsäureamid und 1,74 g Acetessigsäuremethylester in 20 ml Ethanol 2 h unter Rückfluß erhitzt. Nach dem Eindampfen wird der ölige Rückstand an Kieselgel mit Methylenchlorid:Methanol = 95:5 als Elutionsmittel chromatographiert. Man erhält so eine Fraktion, die nach dem Eindampfen 1,53 g Produkt als festen Schaum liefert.

Ausbeute: 1,53 g (32 % d.Th.)        Fp.: 82 - 90°C

Beispiel 12

1,4-Dihydro-2,6-dimethyl-4-/2-(3-pyridyl)ethyl/-pyridin-3,5-dicarbonsäure-3-/1-(ethoxycarbonyl)ethyl/-5-methylester

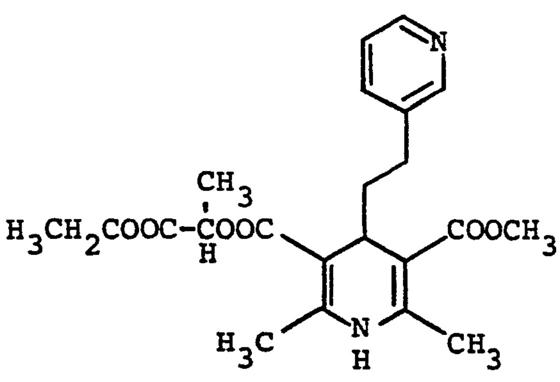

1,1 g 3-(3-Pyridyl)propionaldehyd werden zusammen mit 2 g Acetessigsäure-(1-ethoxycarbonyl)ethylester und 1,15 g 3-Aminocrotonsäuremethylester in 10 ml Ethanol 2 h unter Rückfluß erhitzt. Die Reaktionsmischung wird eingeengt, mit Essigester verdünnt und 2 mal mit 2 n Schwefelsäure extrahiert. Die wäßrige Phase wird mit festem Natriumbicarbonat alkalisch gemacht und mit Methylenchlorid 2 mal extrahiert. Die vereinigten organischen Phasen werden mit Magnesiumsulfat getrocknet und eingedampft. Der Rückstand wird an Kieselgel mit Methylenchlorid/Methanol 95/5 als Elutionsmittel chromatographiert. Man erhält so eine Fraktion, die nach dem Eindampfen aus Ether kristallisiert.

Ausbeute: 3 g (89 % d.Th.)        Fp.: 55 - 60°C

<u>Beispiel 13</u>

1,4-Dihydro-2,6-dimethyl-4-[2-(3-pyridyl)ethyl]-pyridin-3,5-dicarbonsäure-di-3-(3-pyridyl)propylester

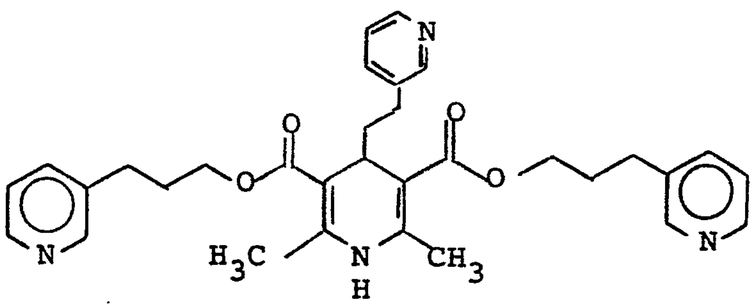

4,55 g Acetessigsäure-[3-(3-pyridyl)]-propylester werden zusammen mit 2,78 g 3-(3-Pyridyl)propionaldehyd und 4,53 g 3-Aminocrotonsäure-[3-(3-pyridyl)]propylester in 50 ml Isopropanol 4 h unter Rückfluß erhitzt. Nach dem Eindampfen im Vakuum erhält man 10,6 g festen Rückstand.

Ausbeute: 10,6 g (95 % d.Th.)        Fp.: 52°C

Beispiel 14

1,4-Dihydro-1,2,6-trimethyl-4-/2-(3-pyridyl)ethyl/-pyridin-3,5-dicarbonsäuredimethylester

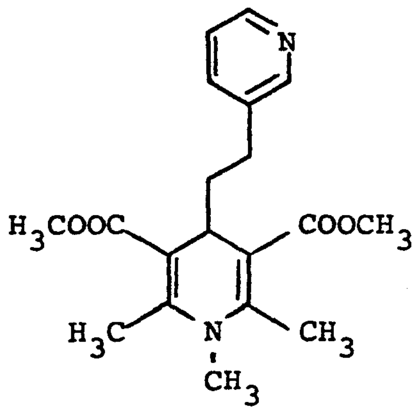

2 g 3-(3-Pyridyl)propionaldehyd, 3,48 g Acetessigsäuremethylester und 1,07 g Methylaminhydrochlorid werden zusammen in 3 ml Pyridin 4 h unter Rückfluß erhitzt. Die Reaktionmischung wird dann mit 150 ml Essigester verdünnt und 3 mal mit Wasser extrahiert. Die organische Phase wird mit Magnesiumsulfat getrocknet und eingedampft. Das als Rückstand erhaltene Öl wird an Kieselgel mit Methylenchlorid:Methanol = 95:5 als Elutionsmittel chromatographiert. Man erhält so eine Fraktion, die nach dem Eindampfen aus Ether kristallisiert.

Ausbeute: 2,5 g (48 % d.Th.)        Fp.: 66 - 72°C.

Beispiel 15

1-Ethoxycarbonylmethyl-1,4-dihydro-2,6-dimethyl-4-/2-(3-pyridyl)ethyl/-pyridin-3,5-dicarbonsäuredimethylester

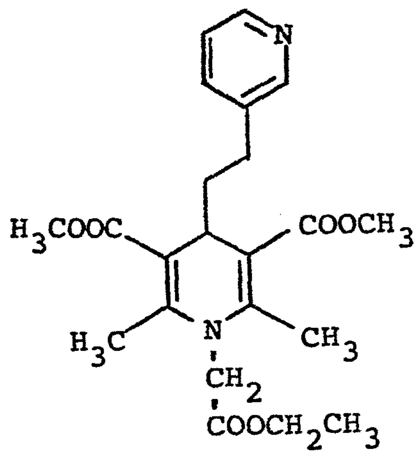

15 g 3-(3-Pyridyl)propionaldehyd werden zusammen mit 25,7 g Acetessigsäuremethylester und 15 g Glycinsäureethylesterhydrochlorid in 20 ml Pyridin bei 90°C 1 h gerührt. Die Reaktionsmischung wird mit 1 n Natronlauge verdünnt und mehrmals mit Essigester extrahiert. Die vereinigten Essigesterphasen werden 1 mal mit ges. Kochsalzlösung gewaschen, mit Magnesiumsulfat getrocknet und eingedampft. Der Rückstand kristallisiert aus einem Gemisch von Ether und Petrolether.

Ausbeute: 37,5 g (82 % d.Th.)        Fp.: 60 - 65°C.

Beispiel 16

1-Carboxymethyl-1,4-dihydro-2,6-dimethyl-4-/2-(3-pyridyl)-ethyl7-pyridin-3,5-dicarbonsäuredimethylester

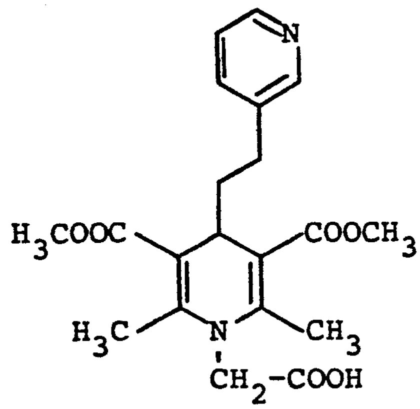

37,5 g 1-Ethoxycarbonylmethyl-1,4-dihydro-2,6-dimethyl-4-/2-(3-pyridyl)ethyl7-pyridin-3,5-dicarbonsäuredimethylester werden in 150 ml Methanol gelöst und mit 55 ml 2 n Natronlauge versetzt. Nach 3 h Rühren bei Raumtemperatur wird mit Wasser verdünnt und mit Methylenchlorid extrahiert. Die wäßrige Phase wird auf einen pH-Wert von 5 gebracht und mehrmals mit Methylenchlorid extrahiert. Die vereinigten organischen Phasen werden mit Magnesiumsulfat getrocknet und eingedampft. Der Rückstand kristallisiert aus Ether.

Ausbeute: 15,5 g (44 % d.Th.) Fp.: 105 - 200°C.

Beispiel 17

1,4-Dihydro-2,6-dimethyl-4-/2-(3-pyridyl)ethyl7-pyridin-3,5-dicarbonsäure-3-(1-carboxy)ethyl-5-methylester

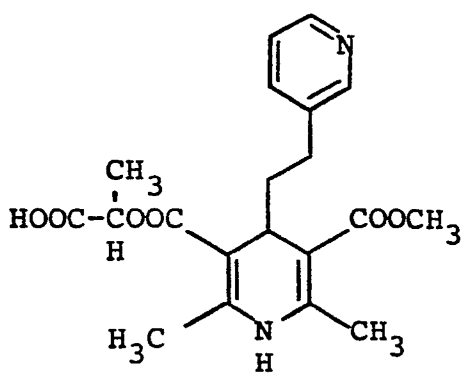

3 g 1,4-Dihydro-2,6-dimethyl-4-/2-(3-pyridyl)ethyl7-pyridin-3,5-dicarbonsäure-3-(1-ethoxycarbonyl)ethyl-5-methylester werden in 20 ml Methanol gelöst und mit 5 ml 40 % Natronlauge versetzt. Nach 2 h Rühren bei Raumtemperatur wird mit Wasser verdünnt, auf pH = 5 gestellt und mit Methylenchlorid 5 mal extrahiert. Die vereinigten org. Phasen werden mit Magnesiumsulfat getrocknet und eingedampft. Der Rückstand kristallisiert aus Ether.

Ausbeute: 1,5 g (54 % d.Th.)        Fp.: 55 - 60°C.

## Beispiel 18

2-Carboxymethyl-1,4-dihydro-6-methyl-4-/2-(3-pyridyl)-ethyl7-pyridin-3,5-dicarbonsäuredimethylester

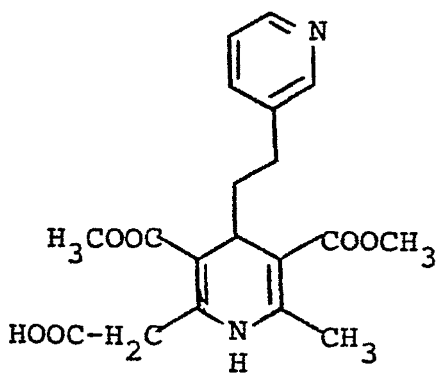

0,7 g 1,4-Dihydro-2-methoxycarbonylmethyl-6-methyl-4-/2-(3-pyridyl)ethyl7-pyridin-3,5-dicarbonsäuredimethylester werden analog Beispiel 16 umgesetzt.

Ausbeute: 0,32 g (48 % d.Th.)        Fp.: 70 - 75°C (Zers.)

Beispiel 19

1,4-Dihydro-2,6-dimethyl-4-[2-(3-pyridyl)ethyl]-pyridin-3,5-dicarbonsäure-5-(3-pyridyl)propylester

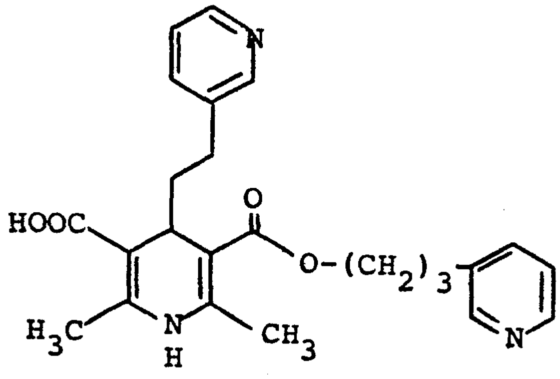

6,75 g 3-(3-Pyridyl)propionaldehyd werden zusammen mit 11,05 g Acetessigsäure-[3-(3-pyridyl)]propylester und 7,7 g 3-Aminocrotonsäure-(2-cyano)ethylester in 100 ml Isopropanol 4 h unter Rückfluß erhitzt. Dann wird die Reaktionslösung im Vakuum eingeengt, in 200 ml Essigester aufgenommen und 2 mal mit 2 n Natronlauge extrahiert. Die organische Phase wird mit Magnesiumsulfat getrocknet und eingedampft. Der Rückstand wird in 100 ml Methanol gelöst und mit 50 ml 2 n Natronlauge versetzt. Nach 12 h Rühren bei Raumtemperatur wird die Reaktionslösung eingedampft, der Rückstand in 200 ml Essigester aufgenommen und 3 mal mit 2 n Natronlauge extrahiert. Die vereinigten Natronlaugephasen werden mit 2 n Schwefelsäure sauer eingestellt und 2 mal mit Essigester gewaschen. Die saure wäßrige Phase wird mit 2 n Natronlauge auf pH = 5,5 gebracht und 3 mal mit Essigester extrahiert. Die vereinigten organischen Phasen werden mit Magnesiumsulfat getrocknet und eingedampft. Der Rückstand kristallisiert aus Ether.

Ausbeute: 12,1 g (51 % d.Th.) Fp.: 50 - 52°C.

Beispiel 20

1,4-Dihydro-2,6-dimethyl-4-/2-(3-pyridyl)ethyl7-pyridin-3,5-dicarbonsäure-5-methylester

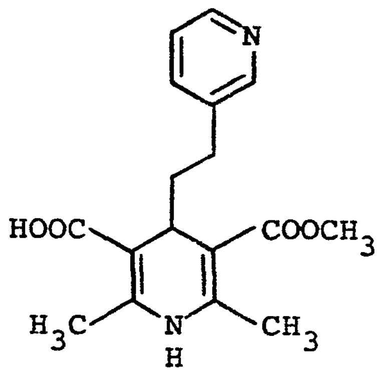

3,25 g 1,4-Dihydro-2,6-dimethyl-4-/2-(3-pyridyl)ethyl7-pyridin-3,5-dicarbonsäure-3-(2-cyano)ethyl-5-methylester werden in 60 ml Methanol gelöst und mit 5,5 ml 2 n Natronlauge versetzt. Nach 16 h Rühren bei Raumtemperatur wird mit 70 ml Wasser verdünnt und 2 mal mit je 80 ml Essigester extrahiert. Die wäßrige Phase wird mit 1 n Salzsäure auf pH = 5,5 gebracht. Das dabei ausfallende Produkt wird abgesaugt und im Vakuum getrocknet.

Ausbeute: 1,6 g (56 % d.Th.) Fp.: 180°C (Zers.)

Beispiel 21

1,4-Dihydro-2,6-dimethyl-4-/2-(3-pyridyl)ethyl7-pyridin-3-carbonsäuremethylester

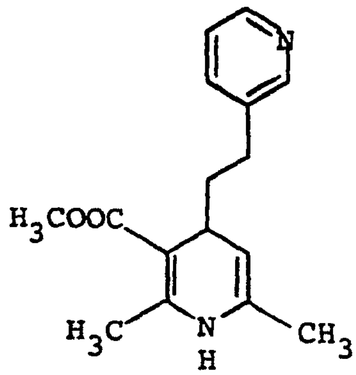

1,5 g 1,4-Dihydro-2,6-dimethyl-4-/2-(3-pyridyl)ethyl7-pyridin-3,5-dicarbonsäure-5-methylester werden in 15 ml Trifluoressigsäure gelöst und 20 h bei 70°C gerührt. Dann wird mit 100 ml Essigester verdünnt und mit 200 ml 2 n Natronlauge (eisgekühlt) extrahiert. Die wäßrige Phase wird 2 mal mit Essigester extrahiert, die vereinigten Essigesterphasen mit Magnesiumsulfat getrocknet und eingedampft. Der Rückstand kristallisiert aus Ether.

Ausbeute: 0,7 g (55 % d.Th.)    Fp.: 55 - 60°C.

Patentansprüche

1. Pyridylethyl-Dihydropyridine der allgemeinen Formel (I)

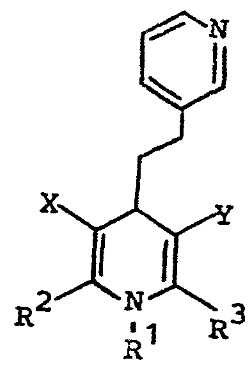

(I)

in welcher

$R^1$ für Wasserstoff oder für geradkettiges oder verzweigtes Alkyl, gegebenenfalls substituiert durch Halogen, Nitro, Cyan, Carboxy, Carbonamido, Alkoxy, Alkoxycarbonyl steht,

$R^2$, $R^3$ gleich oder verschieden sind und für Wasserstoff, für geradkettiges oder verzweigtes Alkyl, gegebenenfalls substituiert durch Halogen, Aryl, Carboxy, Alkoxy, Alkoxycarbonyl stehen oder $R^2$ mit X bzw. $R^3$ mit Y zu einem 5- bis 6-gliedrigen Ring verbunden sind,

X, Y gleich oder verschieden sind und für Wasserstoff, für Cyan oder für die Gruppe $-\underset{\underset{O}{\parallel}}{C}-Z-R^4$

stehen, wobei

Z für eine direkte Bindung oder für Sauerstoff, Schwefel oder NH steht,

und

$R^4$ für Wasserstoff oder für geradkettiges oder verzweigtes Alkyl, gegebenenfalls substituiert durch Halogen, Nitro, Pyridyl, Cyan, Amino, Carboxy, Alkoxy, Alkoxycarbonyl steht,

sowie deren physiologisch unbedenklichen Salze.

2. Pyridylethyl-Dihydropyridine der allgemeinen Formel (I) gemäß Anspruch 1, in welcher

$R^1$ für Wasserstoff oder für geradkettiges oder verzweigtes Alkyl mit bis zu 6 C-Atomen, gegebenenfalls substituiert durch Fluor, Chlor, Brom, Carboxy, Alkoxy oder Alkoxycarbonyl mit jeweils bis zu 6 C-Atomen, steht,

$R^2$, $R^3$ gleich oder verschieden sind und für Wasserstoff oder für geradkettiges oder verzweigtes Alkyl mit bis zu 6 C-Atomen, gegebenenfalls substituiert durch Fluor, Chlor, Brom, Aryl mit 6 bis 10 C-Atomen, Carboxy, Alkoxy, Alkoxycarbonyl mit jeweils bis zu 6 C-Atomen, stehen, oder mit X bzw. Y durch eine Einfachbindung zu einem 5- bis 6-gliedrigen Ring verbunden sind,

X, Y gleich oder verschieden sind und für Wasserstoff für Cyan oder für die Gruppe $-\underset{\underset{O}{\|}}{C}-Z-R^4$

stehen, wobei

Z für eine direkte Bindung oder für Sauerstoff, Schwefel oder NH steht,

und

$R^4$ für Wasserstoff oder für geradkettiges oder verzweigtes Alkyl mit bis zu 6 C-Atomen, gegebenenfalls substituiert durch Fluor, Chlor, Brom, Nitro, Pyridyl, Cyan, Amino, Carboxy, Alkoxy, Alkoxycarbonyl mit jeweils 6 C-Atomen, steht,

sowie deren physiologisch unbedenklichen Salze.

3. Pyridylethyl-Dihydropyridine der allgemeinen Formel (I) gemäß Anspruch 1,

in welcher

$R^1$ für Wasserstoff oder für geradkettiges oder verzweigtes Alkyl mit bis zu 4 C-Atomen, gegebenenfalls substituiert durch Carboxy, Alkoxy, Alkoxycarbonyl mit jeweils bis zu 4 C-Atomen, steht,

$R^2$, $R^3$ gleich oder verschieden sind und Wasserstoff oder für geradkettiges oder verzweigtes Alkyl mit bis zu 4 C-Atomen, gegebenenfalls substituiert durch Fluor, Chlor, Brom, Phenyl, Carboxy, Alkoxycarbonyl mit bis zu 4 C-Atomen, stehen, oder mit X bzw. Y durch eine Einfachbindung zu einem 5- bis 6-gliedrigen Ring verbunden sind,

X, Y gleich oder verschieden sind und für Wasserstoff, für Cyan oder für die Gruppe $-\underset{\overset{\|}{O}}{C}-Z-R^4$ stehen, wobei

Z für eine direkte Bindung oder für Sauerstoff oder NH steht, und

$R^4$ für Wasserstoff oder für geradkettiges oder verzweigtes Alkyl mit bis zu 4 C-Atomen, gegebenenfalls substituiert durch Fluor, Chlor, Brom, Cyan, Pyridyl, Amino, Carboxy, Alkoxy, Alkoxycarbonyl mit jeweils bis zu 4 C-Atomen steht,

sowie deren physiologisch unbedenklichen Salze.

4. Verfahren zur Herstellung der Verbindungen der allgemeinen Formel (I)

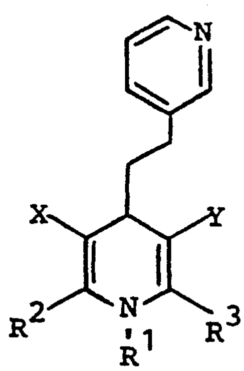

(I)

0173943

in welcher

$R^1$ Wasserstoff oder für geradkettiges oder verzweigtes Alkyl, gegebenenfalls substituiert durch Halogen, Nitro, Cyan, Carboxy, Carbonamido, Alkoxy, Alkoxycarbonyl steht,

$R^2$, $R^3$ gleich oder verschieden sind und für Wasserstoff für geradkettiges oder verzweigtes Alkyl, gegebenenfalls substituiert durch Halogen, Aryl, Carboxy, Alkoxy, Alkoxycarbonyl stehen oder mit X bzw. Y durch eine Einfachbindung zu einem 5- bis 6-gliedrigen Ring verbunden sind,

X, Y gleich oder verschieden sind und für Wasserstoff, für Cyan oder für die Gruppe $-\underset{\overset{\|}{O}}{C}-Z-R^4$

stehen, wobei

Z für eine direkte Bindung oder für Sauerstoff, Schwefel oder NH steht,

und

$R^4$ für Wasserstoff oder für geradkettiges oder verzweigtes Alkyl, gegebenenfalls substituiert durch Halogen, Nitro, Pyridyl, Cyan, Amino, Carboxy, Alkoxy, Alkoxycarbonyl steht,

dadurch gekennzeichnet, daß man

A) Enamine der allgemeinen Formel (II)

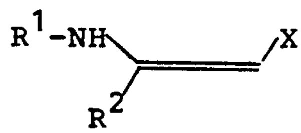

(II)

in welcher

$R^1$, $R^2$, X die oben angegebene Bedetung haben,

X und $R^4$ jedoch nicht für Wasserstoff stehen,

mit Ketoverbindungen der allgemeinen Formel (III)

$$R^3-\underset{\underset{O}{\|}}{C}-CH_2-Y \qquad (III)$$

in welcher

$R^3$, Y die oben angegebene Bedeutung haben,

Y und $R^4$ jedoch nicht für Wasserstoff stehen,

und dem Aldehyd der Formel (IV)

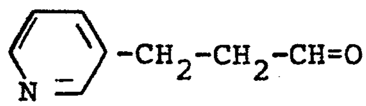

(IV)

gegebenenfalls in Gegenwart eines inerten organischen Lösungsmittels umsetzt,

oder daß man

(B) Ketoverbindungen der allgemeinen Formel III (a, b)

$$R^2\text{-}\underset{\underset{O}{\|}}{C}\text{-}CH_2\text{-}X \quad \text{(IIIa)}, \qquad R^3\text{-}\underset{\underset{O}{\|}}{C}\text{-}CH_2\text{-}Y \quad \text{(IIIb)}$$

gegebenenfalls in Form ihrer Mischungen zu gleichen Teilen falls $R^3$ und $R^2$ voneinander verschieden sind und/oder X und Y voneinander verschieden sind,

in welchen

$R^2$, $R^3$, X, Y die angegebene Bedeutung haben,

X, Y und $R^4$ jedoch nicht für Wasserstoff stehen,

mit dem Aldehyd der Formel (IV) und einem Amin der allgemeinen Formel (V)

$$R^1\text{-}NH_2 \qquad (V)$$

in welcher

$R^1$ die oben angegebene Bedeutung hat, bzw. dessen Salz,

gegebenenfalls in Gegenwart eines inerten organischen Lösungsmittels umsetzt,

und gegebenenfalls Verbindungen der allgemeinen Formel (I), in welchen die Reste $R^1$, $R^2$, $R^3$, X und/oder Y Alkoxycarbonylgruppierungen enthalten und/oder in welchen X oder Y selbst eine Alkoxycarbonylgruppe ist, nach üblichen Methoden verseift und anschließend gegebenenfalls solche, in denen X und Y für Carboxyl steht, decarboxyliert.

5. Verfahren gemäß Anspruch 4, dadurch gekennzeichnet, daß man die Decarboxylierung in saurem Medium durchführt.

6. Verfahren gemäß Anspruch 4, dadurch gekennzeichnet, daß man die Reaktion im Temperaturbereich von 10-150°C durchführt.

7. Arzneimittel enthaltend mindestens eine Verbindung der allgemeinen Formel (I) gemäß den Ansprüchen 1-3.

8. Verbindungen der allgemeinen Formel (I) gemäß den Ansprüchen 1-3 zur Bekämpfung von thromboembolischen und ischämischen Erkrankungen.

9. Verwendung der Verbindungen der allgemeinen Formel (I) gemäß den Ansprüchen 1-3 zur Bekämpfung von Krankheiten.

10. Verfahren zur Herstellung von Arzneimitteln gemäß Anspruch 8, dadurch gekennzeichnet, daß man den oder die Wirkstoffe gegebenenfalls unter Verwendung von üblichen Hilfs- und/oder Trägerstoffen in eine geeignete Applikationsform überführt.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung 0173943

EP 85110718.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 335 466 (BAYER)<br>* Beispiele 8,11,22; Formel I *<br>-- | 1-8,10 | C 07 D 401/06<br>C 07 C 401/14<br>A 61 K 31/44 |
| A | DE - A - 2 005 116 (BAYER)<br>* Formel; Ansprüche 1-3 *<br>-- | 1,7,8,10 | |
| A | CHEMICAL ABSTRACT, Band 87, Nr. 17, 24. Oktober 1977, Columbus, Ohio, USA<br>WHITE, PETER Y.; SUMMERS, LINDSAY A. "(Z)- and (E)-3,3'-(1,2-ethenediyl)bispyridines"<br>Seite 697, Spalte 2, Zusammenfassung-Nr. 134 949w<br>& Aust. J. Chem. 1977, 30(5), 1153-6<br>-- | 1 | |

RECHERCHIERTE SACHGEBIETE (Int. Cl.4)

C 07 D 401/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-8,10

Unvollständig recherchierte Patentansprüche: -

Nicht recherchierte Patentansprüche: 9

Grund für die Beschränkung der Recherche:

Art. 52(4) EPÜ; Verfahren zur therapeutischen Behandlung des menschlichen oder tierischen Körpers

| Recherchenort | Abschlußdatum der Recherche | Prüfer |
|---|---|---|
| WIEN | 15-11-1985 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 08.82

0173943

Nummer der Anmeldung

Europäisches Patentamt

# EUROPÄISCHER TEILRECHERCHENBERICHT

EP 85110718.5

| EINSCHLÄGIGE DOKUMENTE | | | KLASSIFIKATION DER ANMELDUNG (Int. Cl.4) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACT, Band 97, Nr. 6, 9. August 1982, Columbus, Ohio, USA<br>SMITH, PETER A.S.; ROMAINE, JAMES C.; EL-SHEIKH, MUSTAFA "Synthetic mimics for viscosity and spectra of H-coal asphaltenes"<br>Seite 130, Spalte 1, Zusammenfassung-Nr. 41 217x<br>& Prepr. Pap. - Am. Chem. Soc., Div. Fuel Chem. 1980, 25(3), 193-7<br>-- | 1 | |
| | | | TECHNICAL FIELDS SEARCHED (Int. Cl.4) |
| A | CHEMICAL ABSTRACT, Band 96, Nr. 11, 15. März 1982, Columbus, Ohio, USA<br>RYABCHENKO, V.P. "Effect of anabasine hydrochloride on the embryogenesis of white rats and rabbits"<br>Seite 72, Spalte 2, Zusammenfassung-Nr. 79 849x<br>& Farmakol. Toksikol. (Moscow) 1982, 45(1), 87-90<br>-- | 1 | |
| | CHEMICAL ABSTRACT, Band 92, Nr. 23, 9. Juni 1980, Columbus, Ohio, USA<br>VANSANT, J.; SMETS, G.; DECLERCQ, J.P; GERMAIN, G.; VAN MEERSSCHE, M. "Azastilbenes. 1. Synthesis, characterization, and structure"<br>Seite 680, Spalte 1, Zusammenfassung-Nr. 198 354j<br>& J. Org. Chem. 1980, 45(9), 1557-65<br>---- | 1 | |